(19) Europäisches Patentamt
European Patent Office
Office européen des brevets

(11) **EP 2 846 170 A1**

(12) **EUROPEAN PATENT APPLICATION**

(43) Date of publication:
**11.03.2015 Bulletin 2015/11**

(51) Int Cl.:
**G01S 7/52** *(2006.01)* **G01S 15/89** *(2006.01)*

(21) Application number: **14183898.7**

(22) Date of filing: **08.09.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **10.09.2013 JP 2013187153**

(71) Applicant: **Seiko Epson Corporation**
**Tokyo 163 (JP)**

(72) Inventor: **Hayashi, Masaki**
**Suwa-shi, Nagano 392-8502 (JP)**

(74) Representative: **Hoffmann Eitle**
**Patent- und Rechtsanwälte PartmbB**
**Arabellastraße 30**
**81925 München (DE)**

(54) **Ultrasonic measurement apparatus, ultrasonic imaging apparatus, and ultrasonic measurement method**

(57)    An ultrasonic measurement apparatus that is able to prevent a decrease in the effects of adaptive beamforming, in the case where some element cannot correctly receive signals. The ultrasonic measurement apparatus has a reception processing unit that receives, via an ultrasonic element array having a plurality of channels, an ultrasonic echo relating to an ultrasonic wave transmitted toward an object, as a reception signal for each channel, an error detection unit that performs error detection for each channel (S111), a signal processing unit that performs weighted addition of a reception signal for each normal channel other than an error channel for which an error was detected, with a weight that depends on the reception signal for the normal channel (S121), and an image generation unit that generates an image based on a signal obtained from the weighted addition.

FIG.11

EP 2 846 170 A1

**Description**

BACKGROUND

1. Technical Field

The present invention relates to an ultrasonic measurement apparatus, an ultrasonic imaging apparatus, and an ultrasonic measurement method.

2. Related Art

Technologies for performing adaptive beamforming on reception waves of ultrasonic echoes relating to a transmitted ultrasonic wave in an ultrasonic measurement apparatus are known. Adaptive beamforming is able to maximize the sensitivity of reception waves from a desired direction and to minimize the sensitivity of unwanted waves from directions other than the desired direction. That is, the sensitivity characteristics of reception waves can be adaptively changed and azimuth resolution can be improved.

As an example of adaptive beamforming, JP-A-2012-170826 discloses an ultrasonic beamforming method that divides an input ultrasonic wave signal into a plurality of regions in observation space, calculates weighted values by region, calculates pixel weighted values for respective pixels, and calculates a beamforming value.

However, in the case where signals cannot be correctly received by some of the ultrasonic transducer elements that perform ultrasonic wave reception, there is a problem in that the effects of adaptive beamforming cannot be obtained due to not being able to correctly derive the sensitivity characteristics. An element cannot correctly receive a signal in cases such as where, for example, a fault occurs in the element itself, a circuit that controls the element or the like, or where obstacles that reflect the ultrasonic wave such as air bubbles exist between the element and the subject.

SUMMARY

An advantage of some aspects of the invention is to provide a technology that prevents a decrease in the effects of adaptive beamforming, even in the case where some of the elements cannot correctly receive signals.

An ultrasonic measurement apparatus according to a first aspect of the invention includes a reception processing unit that receives, via an ultrasonic element array having a plurality of channels, an ultrasonic echo relating to an ultrasonic wave transmitted toward an object, as a reception signal for each channel; an error detection unit that performs error detection for each channel; a signal processing unit that performs weighted addition of a reception signal for each normal channel other than an error channel for which an error was detected, with a weight that depends on the reception signal for the normal channel; and an image generation unit that generates an image based on a signal obtained from the weighted addition. According to the first aspect of the invention, an error channel is detected and weighted addition processing on the reception signals of normal channels other than the error channel is executed according to the reception signal of each normal channel. Even in the case where there is an error channel, the influence of the reception signal of the error channel are thereby avoided, and a decrease in the effects of weighted addition processing such as adaptive beamforming can be prevented.

The reception processing unit may receive an ultrasonic echo relating to the transmitted ultrasonic wave as the reception signal for each channel, whenever an ultrasonic wave for generating an image for one line is transmitted, the error detection unit may perform error detection for each channel, whenever the reception signal for each channel is received, the signal processing unit may perform weighted addition, whenever the reception signal for each channel is received, and the image generation unit may generate an image, whenever the reception signal for each channel is received. A fault or the like that occurs due to aged deterioration of individual channels or the like can thereby be accurately detected. Also, since adaptive beamforming is executed on the reception signals of normal channels for each line, the likelihood of a decrease in the effects of adaptive beamforming can be reduced.

The error detection unit may detect a channel whose reception signal has a relatively low value as an error channel, based on a value of the received reception signal for each channel. Error channels can thereby be accurately detected, even if aged deterioration or the like occurs in all the channels.

The error detection unit may detect a channel whose reception signal has a relatively low value as an error channel, based on a value of a reflection signal from a specific object in an ultrasonic probe that includes the ultrasonic element array, among the received reception signals for the respective channels. Error channels can thereby be accurately detected, without being affected by the external environment or the like.

The error detection unit may detect a channel whose reception signal has a value less than a predetermined value as an error channel, based on a value of the received reception signal for each channel. Operational processing for performing relative comparison with other channels is thereby no longer necessary, enabling the processing time of error detection to be reduced.

The signal-processing unit may determine whether the number of the detected error channels is less than or equal to a predetermined number, perform weighted addition of the reception signal for each normal channel with the weight that depends on the reception signal for the normal channel, if the number of detected error channels is less than or equal to the predetermined number, and perform weighted addition of the reception signal for each error channel and each normal channel with a predetermined fixed weight, if the number of detected error channels is not less than or equal to the predetermined number. Resolution can thereby be improved by executing normal beamforming, in the case where resolution would conversely decrease if weighted addition such as adaptive beamforming were executed.

The signal processing unit may derive, as the weight that depends on the reception signal, a weight of each normal channel at which a variance of a value obtained by multiplying a weight of the normal channel and the reception signal of the normal channel is minimized. The weight of each channel can thereby be changed according to the incoming wave.

An ultrasonic imaging apparatus according to a second aspect of the invention includes a reception processing unit that receives, via an ultrasonic element array having a plurality of channels, an ultrasonic echo relating to an ultrasonic wave transmitted toward an object, as a reception signal for each channel; an error detection unit that performs error detection for each channel; a signal processing unit that performs weighted addition of a reception signal for each normal channel other than an error channel for which an error was detected, with a weight that depends on the reception signal for the normal channel; an image generation unit that generates an image based on a signal obtained from the weighted addition; and a display unit that displays the generated image. Even in the case where there is an error channel, the influence of the reception signal of the error channel are thereby avoided, and a decrease in the effects of weighted addition processing such as adaptive beamforming can be prevented. As a result, an ultrasonic image with improved azimuth resolution can be obtained.

An ultrasonic measurement method according to a third aspect of the invention includes receiving, via an ultrasonic element array having a plurality of channels, an ultrasonic echo relating to an ultrasonic wave transmitted toward an object, as a reception signal for each channel; performing error detection for each channel; performing weighted addition of a reception signal for each normal channel other than an error channel for which an error was detected, with a weight that depends on the reception signal for the normal channel; and generating an image based on a signal obtained from the weighted addition. Even in the case where there is an error channel, the influence of the reception signal of the error channel is thereby avoided, and a decrease in the effects of weighted addition processing such as adaptive beamforming can be prevented.

BRIEF DESCRIPTION OF THE DRAWINGS

The invention will be described with reference to the accompanying drawings, wherein like numbers reference like elements.

Fig. 1 shows an exemplary external appearance of an ultrasonic imaging apparatus according to an embodiment of the invention.
Fig. 2 shows an exemplary configuration of an ultrasonic transducer element.
Fig. 3 shows an exemplary configuration of an ultrasonic transducer device (element chip).
Figs. 4A and 4B show an exemplary configuration of ultrasonic transducer element groups UG (UG1 to UG64), with Fig. 4A showing the case where there are four element columns, and Fig. 4B showing the case where there is one element column.
Fig. 5 is a block diagram showing an exemplary functional configuration of a control unit.
Fig. 6 illustrates an exemplary structure of data relating to each channel that is stored in a memory.
Fig. 7 shows an exemplary hardware configuration for realizing the functions of the control unit.
Fig. 8 illustrates a signal delay at each channel.
Fig. 9 illustrates sub apertures in spatial averaging.
Fig. 10 is a flowchart (1/4) showing exemplary processing that is realized by the ultrasonic imaging apparatus.
Fig. 11 is a flowchart (2/4) showing exemplary processing that is realized by the ultrasonic imaging apparatus.
Fig. 12 is a flowchart (3/4) showing exemplary processing that is realized by the ultrasonic imaging apparatus.
Fig. 13 is a flowchart (4/4) showing exemplary processing that is realized by the ultrasonic imaging apparatus.

DESCRIPTION OF EXEMPLARY EMBODIMENTS

Hereinafter, embodiments of the invention will now be described with reference to the drawings.

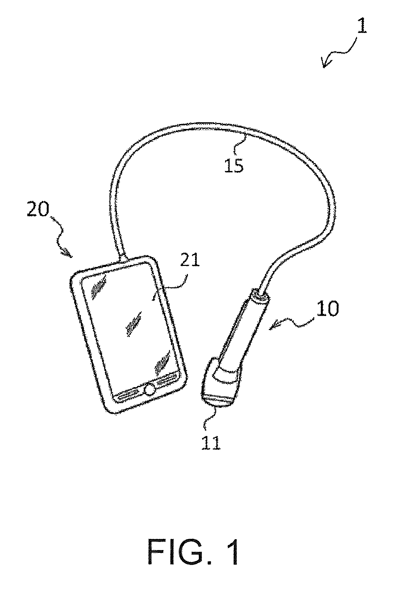

Fig. 1 shows an exemplary external appearance of an ultrasonic imaging apparatus 1 according to an embodiment of the invention. The ultrasonic imaging apparatus 1 is, for example, a compact apparatus, and has an ultrasonic probe 10 and an ultrasonic imaging apparatus main body 20. The ultrasonic probe 10 and the ultrasonic imaging apparatus main body 20 are connected by a cable 15. Note that the ultrasonic imaging apparatus 1 is not limited to being a compact apparatus, and may be, for example, a stationary apparatus, or an integrated apparatus in which the ultrasonic probe is built into the apparatus main body.

The ultrasonic probe 10 has an ultrasonic transducer device 11. The ultrasonic transducer device 11 transmits an ultrasonic beam toward an object while scanning over the object along a scan surface, and receives ultrasonic echoes resulting from the ultrasonic beam.

Also, the ultrasonic probe 10 has an acoustic matching layer and an acoustic lens (both not shown) on the side of the ultrasonic transducer device 11 on which ultrasonic waves are transmitted and received. The acoustic matching layer is a member for reducing reflection of an ultrasonic wave from the surface of the object and allowing the ultrasonic wave to be incident on the object in an efficient manner. The acoustic lens is a member for preventing the ultrasonic beam emitted from the ultrasonic transducer device 11 from spreading, and for causing the ultrasonic beam to converge in the slice direction.

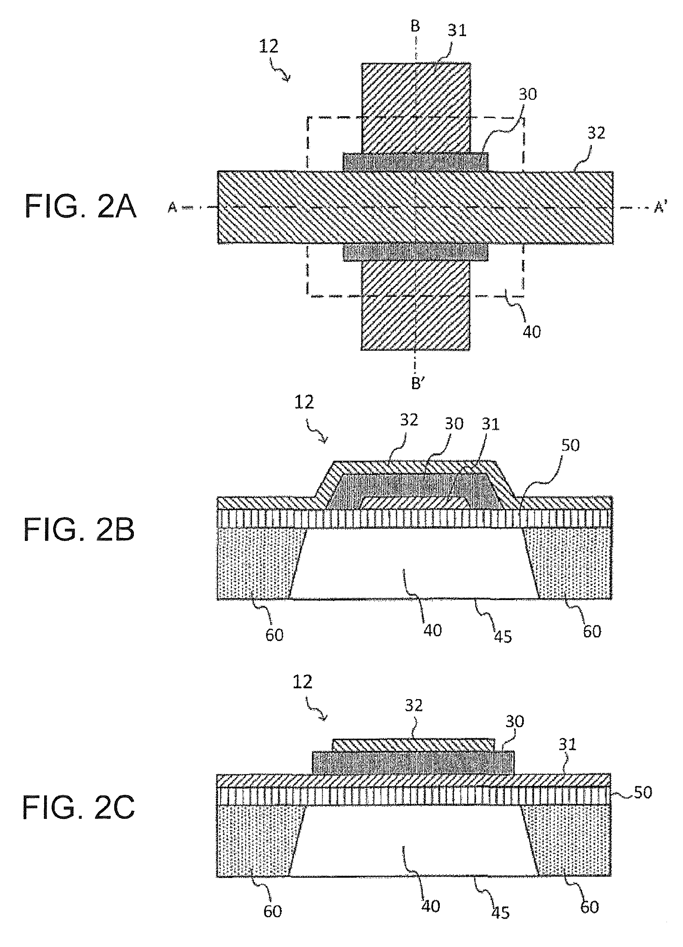

Taking a type that uses piezoelectric elements as an example, the ultrasonic transducer device 11 has a plurality of ultrasonic transducer elements 12 (ultrasonic element array; refer to Fig. 2, etc.) and a substrate in which a plurality of apertures are disposed in an array.

Fig. 2 shows an exemplary configuration of an ultrasonic transducer element. In the present embodiment, a monomorph (unimorph) structure in which a thin piezoelectric element and a metal plate (vibration film) are stuck together is employed as the ultrasonic transducer elements 12.

Fig. 2A is a plan view of an ultrasonic transducer element 12 formed on a substrate (silicon substrate) 60 viewed from an element formation side in a direction perpendicular to a substrate 60. Fig. 2B is a cross-sectional view showing a cross-section along A-A' in Fig. 2A. Fig. 2C is a cross-sectional view showing a cross-section along B-B' in Fig. 2A.

The ultrasonic transducer element 12 has a piezoelectric element part and a vibration film (membrane, supporting member) 50. The piezoelectric element part has a piezoelectric layer (piezoelectric film) 30, a first electrode layer (lower electrode) 31, and a second electrode layer (upper electrode) 32.

The piezoelectric layer 30 is formed using a PZT (lead zirconate titanate) thin film, for example, and is provided so as to cover at least a portion of the first electrode layer 31. Note that the material of the piezoelectric layer 30 is not limited to PZT, and materials such as lead titanate ($PbTiO_3$), lead zirconate ($PbZrO_3$) and lead lanthanum titanate (($Pb$, $La$)$TiO_3$), for example, may be used.

The first electrode layer 31 is formed on an upper layer of the vibration film 50 with a metal thin film, for example. This first electrode layer 31 may be an interconnect that extends to outside the element formation area as shown in Fig. 2A, and is connected to an adjacent ultrasonic transducer element 12.

The second electrode layer 32 is formed with a metal thin film, for example, and is provided so as to cover at least a portion of the piezoelectric layer 30. This second electrode layer 32 may be an interconnect that extends to outside the element formation area as shown in Fig. 2A, and is connected to an adjacent ultrasonic transducer element 12.

The lower electrode (first electrode) of the ultrasonic transducer element 12 is formed by the first electrode layer 31, and the upper electrode (second electrode) is formed by the second electrode layer 32. Specifically, the portion of the first electrode layer 31 covered by the piezoelectric layer 30 forms the lower electrode, and the portion of the second electrode layer 32 covering the piezoelectric layer 30 forms the upper electrode. That is, the piezoelectric layer 30 is provided so as to be sandwiched between the lower electrode and the upper electrode.

An aperture 40 is formed by etching such as reactive ion etching (RIE) or the like from the back surface (surface on which the element is not formed) side of the substrate 60 (silicon substrate). The resonance frequency of ultrasonic waves is determined by the size of the aperture 40, with the ultrasonic waves being emitted to the piezoelectric layer 30 side (in a direction from far to near in Fig. 2A).

The vibration film (membrane) 50 is provided so as to close the aperture 40 using a two layer structure consisting of a $SiO_2$ thin film and a $ZrO_2$ thin film, for example. This vibration film 50 supports the piezoelectric layer 30 and the first and second electrode layers 31 and 32, and produces ultrasonic waves by vibrating in accordance with the expansion and contraction of the piezoelectric layer 30.

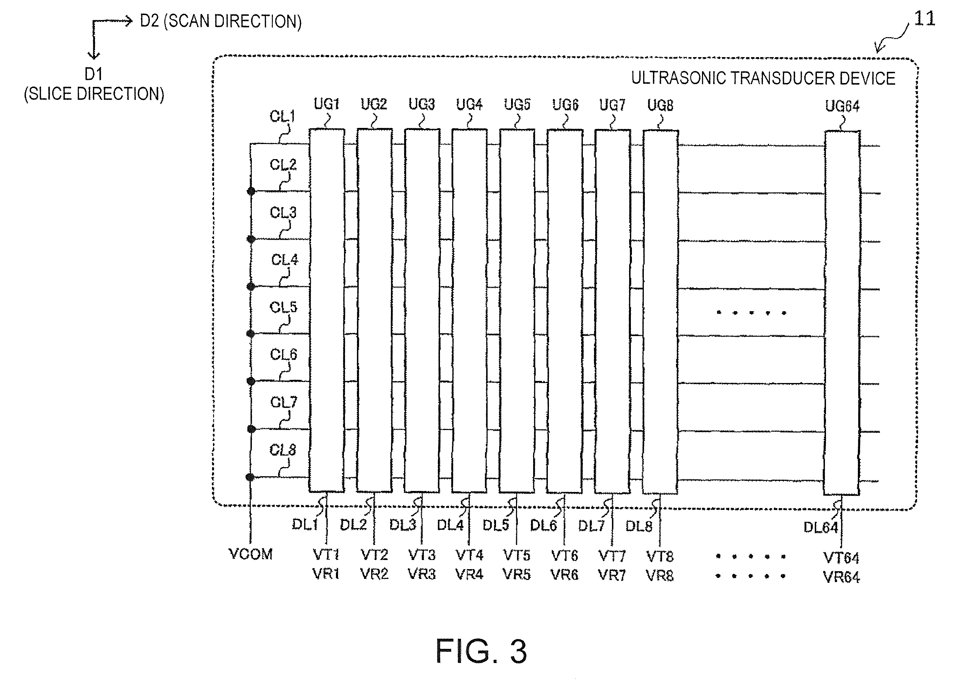

Fig. 3 shows an exemplary configuration of the ultrasonic transducer device (element chip). The ultrasonic transducer device 11 of this exemplary configuration includes a plurality of ultrasonic transducer element groups UG1 to UG64 and drive electrode lines DL1 to DL64 (broadly, 1st to mth drive electrode lines, where m is an integer of 2 or more) and common electrode lines CL1 to CL8 (broadly, 1st to nth common electrode lines, where n is an integer of 2 or more). Note that the number (m) of drive electrode lines and the number (n) of common electrode lines are not limited to the numbers shown in Fig. 3.

The plurality of ultrasonic transducer element groups UG1 to UG64 are disposed in 64 columns in a second direction D2 (scan direction). Each of the ultrasonic transducer element groups UG1 to UG64 has a plurality of ultrasonic transducer elements that are disposed in a first direction D1 (slice direction).

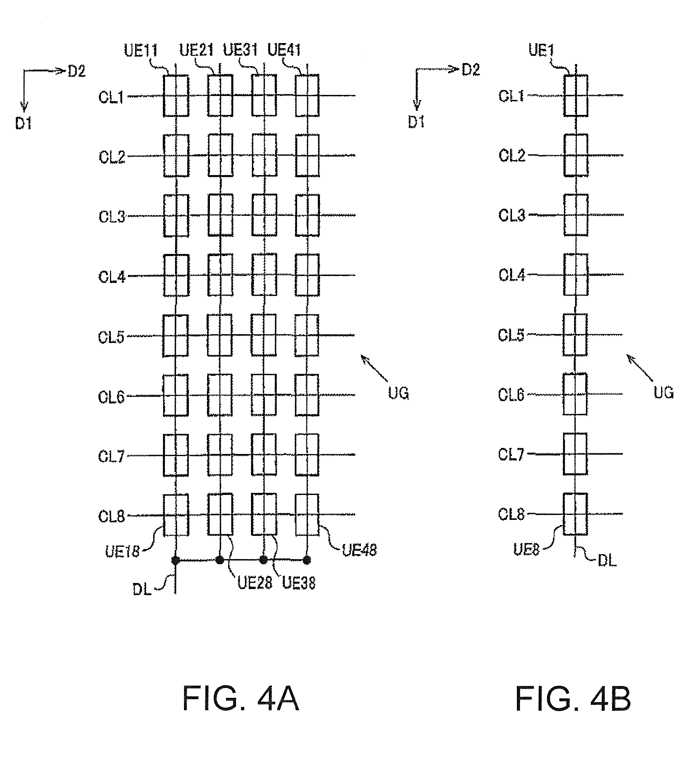

Fig. 4A shows an exemplary ultrasonic transducer element group UG (UG1 to UG64). In Fig. 4A, the ultrasonic transducer element group UG is constituted by first to fourth element columns. The first element column is constituted by ultrasonic transducer elements UE11 to UE18 that are disposed in the first direction D1, and the second element column is constituted by ultrasonic transducer elements UE21 to UE28 that are disposed in the first direction D1. The third element column (UE31 to UE38) and the fourth element column (UE41 to UE48) are also similarly constituted. The drive electrode line DL (DL1 to DL64) is commonly connected to the first to fourth element columns. Also, the common electrode lines CL1 to CL8 are connected to the ultrasonic transducer elements of the first to fourth element columns.

The ultrasonic transducer element group UG in Fig. 4A constitutes one channel of the ultrasonic transducer device. That is, the drive electrode line DL is equivalent to the drive electrode line of one channel, and the transmission signal of one channel from a transmission circuit is input to the drive electrode line DL. Also, the reception signal of one channel constituted by the ultrasonic transducer element group UG is output from the drive electrode line DL. Note that the number of element columns constituting one channel is not limited to four columns as shown in Fig. 4A, and may be less than four columns or greater than four columns. For example, one channel may be constituted by a single element column, as shown in Fig. 4B.

Returning to the description of Fig. 3, the drive electrode lines DL1 to DL64 (1st to mth drive electrode lines) are laid in the first direction D1. An ith drive electrode line DLi (ith channel) of the drive electrode lines DL1 to DL64 (where i is an integer such that $1 \leq i \leq m$) is connected to the first electrode (e.g., lower electrode) of the ultrasonic transducer elements of the ith ultrasonic transducer element group UGi.

Transmission signals VT1 to VT64 are supplied to the ultrasonic transducer elements UE via the drive electrode lines DL1 to DL64 in a transmission period for emitting ultrasonic waves. Also, reception signals VR1 to VR64 from the ultrasonic transducer elements UE are output via the drive electrode lines DL1 to DL64 in a reception period for receiving ultrasonic echo signals.

The common electrode lines CL1 to CL8 (1st to nth common electrode lines) are laid in the second direction D2. The second electrode of the ultrasonic transducer elements is connected to one of the common electrode lines CL1 to CL8. Specifically, as shown in Fig. 3, for example, a jth common electrode line CLj (where j is an integer such that $1 \leq j \leq n$) of the common electrode lines CL1 to CL8 is connected to the second electrode (e.g., upper electrode) of the ultrasonic transducer elements that are disposed in the jth line.

A common voltage $V_{COM}$ is supplied to the common electrode lines CL1 to CL8. This common voltage $V_{COM}$ need only be a constant direct current voltage, and not 0V, that is, not ground potential.

In the transmission period, a difference voltage between the transmission signal voltage and the common voltage is applied to the ultrasonic transducer elements, and ultrasonic waves of a predetermined frequency are emitted.

Note that the arrangement of the ultrasonic transducer elements is not limited to the matrix arrangement shown in Fig. 3, and may be in a so-called houndstooth arrangement in which the elements of any two adjacent columns are disposed so as to zigzag alternately. Also, in Figs. 4A and 4B, the case is shown where a single ultrasonic transducer element is used as both a transmission element and a reception element, but the present embodiment is not limited thereto. For example, ultrasonic transducer elements for use as transmission elements and ultrasonic transducer elements for use as reception elements may be provided separately, and disposed in an array.

Also, the ultrasonic transducer elements 12 are not limited to a configuration that uses piezoelectric elements. For example, transducers that use capacitive elements, such as capacitive micro-machined ultrasonic transducers (cMUTs) may be employed, or bulk transducers may be employed.

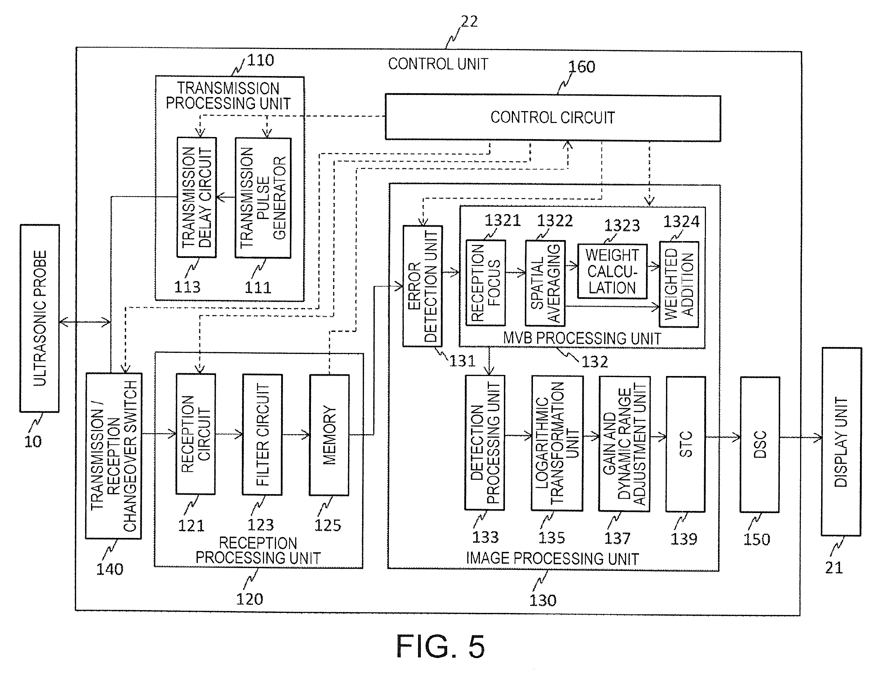

Returning to the description of Fig. 1, a display unit 21 is provided in the ultrasonic imaging apparatus main body 20. The display unit 21 displays image data for display generated by a control unit 22 (refer to Fig. 5) provided in the ultrasonic imaging apparatus main body 20. A display device such as a liquid crystal display, an organic electroluminescence display or electronic paper, for example, can be used for the display unit 21.

Fig. 5 is a block diagram showing an exemplary functional configuration of the control unit 22. The control unit 22 has a transmission processing unit 110, a reception processing unit 120, an image processing unit 130, a transmission/reception changeover switch 140, a digital scan converter (DSC) 150, and a control circuit 160. Note that, in the present embodiment, the control unit 22 is provided in the ultrasonic imaging apparatus main body 20, but at least a portion of the configuration of the control unit 22 may be provided in the ultrasonic probe 10.

The transmission processing unit 110 performs processing for transmitting ultrasonic waves toward the object. The transmission processing unit 110 has a transmission pulse generator 111 and a transmission delay circuit 113.

The transmission pulse generator 111 applies a transmission pulse voltage to drive the ultrasonic probe 10.

The transmission delay circuit 113 performs transmission focusing control, and causes the ultrasonic probe 10 to emit an ultrasonic beam corresponding to the generated pulse voltage toward the object. Thus, the transmission delay circuit 113 provides a time difference between channels with regard to the application timing of the transmission pulse voltage, and causes the ultrasonic waves produced by the plurality of vibration elements to converge. It is thus possible to arbitrarily change the focal length by changing the delay time.

In the case of linear scanning, the full aperture (64 channels in the example shown in Fig. 3) is divided, and transmission and reception is performed with the resultant aperture (use aperture), with individual lines being generated while shifting the use aperture. The use apertures can be set to eight channels, for example. Note that beam width narrows and azimuth resolution increases with enlargement of the use aperture. In the case of sector scanning, the full aperture is used as the use aperture, and individual lines are generated while changing the beam direction.

The transmission/reception changeover switch 140 performs changeover processing of ultrasonic wave transmission and reception. The transmission/reception changeover switch 140 protects the reception processing unit 120 from input of amplitude pulses at the time of transmission, and allows signals at the time of reception to pass through to the reception processing unit 120.

The reception processing unit 120 performs processing for receiving reception waves of ultrasonic echoes relating to transmitted ultrasonic waves (hereinafter, reception waves). The reception processing unit 120 has a reception circuit 121, a filter circuit 123, and a memory 125.

The reception circuit 121 converts the reception wave (analog signal) for each channel into a digital reception signal, and outputs the reception signal to the filter circuit 123. Note that focusing control of the reception waves is performed with the image processing unit 130 which will be discussed later.

The filter circuit 123 performs filtering with a bandpass filter or the like on the reception signal for each channel output from the receiving circuit 121, and removes noise. The filter circuit 123 then outputs the reception signal for each channel to which filtering was applied to the memory 125.

The memory 125 stores, for each channel, the reception signal for the channel output from the filter circuit 123. The functions of the memory 125 can be realized by using a storage device such as a random access memory (RAM).

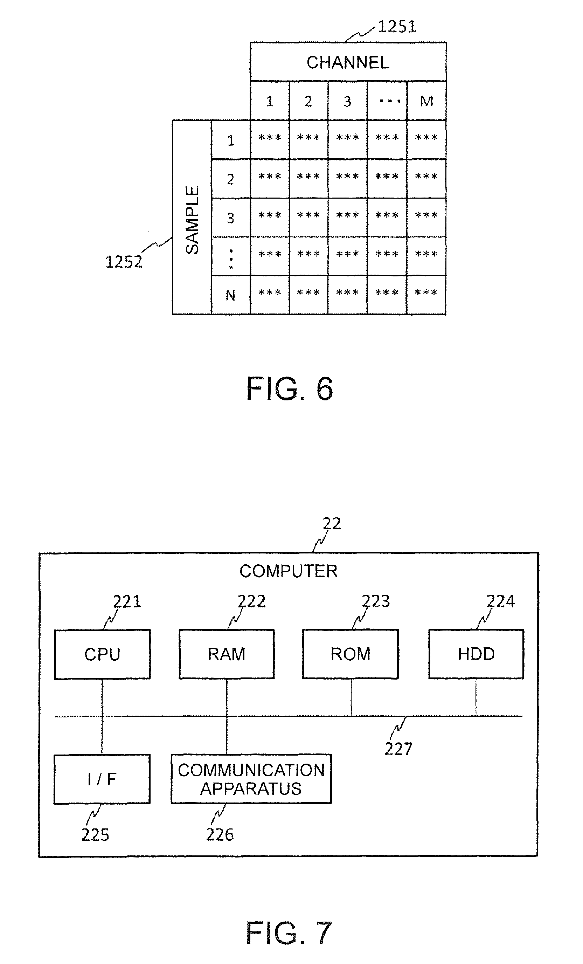

Fig. 6 illustrates an exemplary structure of the data relating to each channel that is stored in the memory. The memory 125 stores, for each of the M channels, the transmission of one ultrasonic wave and the reception waves of ultrasonic echoes relating thereto as waveform data of N sample reception signals. Note that M is the total number of channels of the use aperture and N is the total number of samplings. The total number of samplings is determined by, for example, a prescribed sampling frequency (e.g., 50 MHz) of the ultrasonic imaging apparatus 1, and the observation time for one reception wave.

Returning to the description of Fig. 5, the functions of the reception processing unit 120 can be realized by, for example, an analog front end (AFE) that is constituted by a low noise amplifier (LNA), a programmable gain amplifier (PGA), a filter circuit, an analog/digital converter (A/D convertor), and the like.

Note that the configuration of the reception processing unit 120 is not restricted to the illustrated example. For example, the filter circuit 123 may be provided in the image processing unit 130 or upstream of an MVB processing unit 132, and filtering may be performed on the reception signal for each channel. In this case, the functions of a filtering circuit may be realized by software.

The image processing unit 130 acquires the reception signals stored in the memory 125 of the reception processing unit 120, and performs various image processing. The image processing unit 130 has an error detection unit 131, the minimum variance beamforming (MVB) processing unit 132, a detection processing unit 133, a logarithmic transformation unit 135, a gain and dynamic range adjustment unit 137, and a sensitivity time control (STC) 139. Note that the MVB processing unit may also be referred to as a signal processing unit. Also, the function relating to image generation among the functions of the image processing unit 130 (realized by detection processing unit 133, logarithmic transformation unit 135, gain and dynamic range adjustment unit 137, and STC 139) may be referred to as an image generation unit.

The error detection unit 131 performs error detection on each channel, based on the reception signal for the channel stored in the memory 125. The error detection unit 131 will be discussed in detail later.

The MVB processing unit 132 performs MVB processing, which is directionally-constrained adaptive beamforming, based on the reception signals of channels (hereinafter, "normal channels") other than channels (hereinafter, "error channels") for which an error was detected by the error detection unit 131, among the reception signals for the channels stored in the memory 125. The MVB processing unit 132 performs normal beamforming processing, however, in the case where there are more than a predetermined number of error channels. Thus, the MVB processing unit 132 has a reception focus processing unit 1321, a spatial averaging processing unit 1322, a weight calculation unit 1323, and a weighted addition unit 1324. The MVB processing unit 132 will be discussed in detail later.

The detection processing unit 133 performs absolute value (rectification) processing on the reception signals that have undergone MVB processing or normal beamforming processing, and applies a low-pass filter to extract an unmodulated signal.

The logarithmic transformation unit 135 performs Log compression on the extracted unmodulated signal, and converts the form of expression of the signal, so as to more easily confirm the maximum and minimum signal strengths of reception signals at the same time.

The gain and dynamic range adjustment unit 137 adjusts the signal strength and the area of interest. For example, in gain adjustment processing, a direct current component is added to the Log-compressed input signal. Also, in dynamic range adjustment processing, the Log-compressed input signal is multiplied by an arbitrary number.

The STC 139 corrects the degree of amplification (brightness) according to depth, and acquires an image having uniform brightness across the entire screen.

Note that the functions of the image processing unit 130 can be realized by hardware such as various processors (CPU, etc.), an ASIC (gate array, etc.) or the like, computer programs, or the like.

The DSC 150 performs scan conversion on B-mode image data. For example, the DSC 150 converts line signals into image signals by interpolation processing such as bilinear interpolation. The DSC 150 then outputs the image signals to the display unit 21. Images are thereby displayed on the display unit 21.

The control circuit 160 performs control of the transmission pulse generator 111, the transmission delay circuit 113, the transmission/reception changeover switch 140, the reception circuit 121, the memory 125, the MVB processing unit 131, and the like.

Although the main configuration of the ultrasonic imaging apparatus 1 has been described above in describing the features of the present embodiment, the configuration of the ultrasonic imaging apparatus 1 is not limited to the above configuration. The instant invention is not restricted by the classification method or names of the constituent elements. The configuration of the ultrasonic imaging apparatus 1 can also be classified into more constituent elements according to the processing content. One constituent element can also be classified so as to execute more processing. Also, the processing of each constituent element may be executed by one piece of hardware or may be executed by multiple pieces of hardware.

Fig. 7 shows an exemplary hardware configuration for realizing the functions of the control unit. As shown in Fig. 7, the control unit 22 can be realized by a computer that is provided with, for example, a central processing unit (CPU) 221 which is an arithmetic device, a random access memory (RAM) 222 which is a volatile storage device, a read only memory (ROM) 223 which is a nonvolatile storage device, a hard disk drive (HDD) 224, an interface (I/F) circuit 225 that connects the control unit 22 with other units, a communication apparatus 226 that performs communication with external devices, and a bus 227 that connects these constituent elements with each other.

At least some of the above functions of the control unit 22 are realized by the CPU 221 reading out predetermined programs stored in the ROM 223 or the HDD 224 to the RAM 222 and executing the read programs. Note that the predetermined programs may, for example, be installed in the ROM 223 or the HDD 224 in advance, or may be downloaded from a network via the communication apparatus 226 and installed or updated.

Next, the error detection unit 131 will be described.

As mentioned above, the ultrasonic probe 10 is provided with an acoustic matching layer and an acoustic lens, in addition to the ultrasonic transducer device 11. Here, an ultrasonic wave transmitted from the ultrasonic transducer device 11 is reflected by not only the object but also by members within the ultrasonic probe 10 such as the acoustic lens. That is, each channel of the ultrasonic transducer device 11 receives not only reflected waves from the object but also reflected wave from members such as the acoustic lens.

In view of this, in the present embodiment, error channel judgment is performed based on the reception signals of reflected waves from the acoustic lens. For example, when the distance from the transducer element to the acoustic lens is d (e.g., 2 mm), and the sound velocity is c (e.g., 1000 m/s), a reflected wave will reach each channel in 2 d/c $\mu$s after transmission of an ultrasonic wave from the channel. Since the time interval in which the reflected wave from the acoustic lens will be received by each channel is thus known, data for a period corresponding to the reflected wave from the acoustic lens can be specified and acquired from the data of each sampling point of the reception signal of each channel stored in the memory 125.

Here, assume that the total number of channels of the use aperture is M, and the total number of samplings is N, as illustrated in Fig. 6. Also, the reception signal of the mth (1 to M) channel is expressed as $x_m$, and the reception signal in the sampling point n (1 to N) of the mth channel is expressed as $x_m[n]$. Also, the period (sampling points) corresponding to the reflected wave from the acoustic lens is given sample numbers 1 to 100 (where N is sufficiently larger than 100).

The error detection unit 131 computes, for each channel, a total value $S_m$ of data for the period corresponding to the reflected wave from the acoustic lens by the following equation (1).

$$S_m = \sum_{n=1}^{100} \left| x_m[n] \right| \qquad \cdots (1)$$

Also, the error detection unit 131 computes a standard deviation $\sigma$ between the channels, based on the total value $S_m$ of each channel, using the following equation (2).

$$\sigma = \pm \sqrt{\frac{1}{m} \sum_{i=1}^{m} \left( S_i - \bar{S} \right)^2} \qquad \cdots (2)$$

The error detection unit 131 then determines, on the basis of the average value of the total values $S_m$ of the channels, whether the total value $S_m$ of each channel is more than $2\sigma$ above the average value or more than $2\sigma$ below the average value, as shown in equation (3). If the total value $S_m$ is more than $2\sigma$ above or below the average value, the error detection unit 131 judges that the channel is an error channel. Note that the error range is not limited to a range of $2\sigma$, and may be set to another range such as $3\sigma$.

$$S_m > \bar{S} + 2\sigma \quad or \quad S_m < \bar{S} - 2\sigma \qquad \cdots (3)$$

The error detection unit 131 is thereby able to judge that a channel whose signal of the reflected wave from the acoustic lens is large or small is in error, with respect to a relative reference value that is determined by the signal of the reflected wave from the acoustic lens for each channel. This method enables mainly faults in channels and the like to be detected. Note that the member of the ultrasonic probe 10 that is used is not limited to the acoustic lens. Error channels may also be specified, based on the reflected waves from an object that is external to the ultrasonic probe 10 rather than being limited to a member provided inside the ultrasonic probe 10.

It should be obvious that the method of error detection is not limited to the above method. For example, although error judgment is based on a relative reference in the above method, error judgment may be based on an absolute reference. Specifically, since the acoustic impedances of the acoustic matching layer and the acoustic lens are known, the reflected intensity (reflectance) of the acoustic lens with respect to an ultrasonic wave can be derived in advance. The error detection unit 131 derives, for each channel, the total value of data for a period corresponding to the reflected wave from the acoustic lens, and determines whether the total value is less than a predetermined value set based on the reflected intensity (e.g., value obtained by multiplying the intensity of the transmission wave by the reflectance and adjusting the resultant value by a coefficient, etc.). If the signal of the reflected wave from the acoustic lens is less than a predetermined value, the channel can be judged to be in error. This method also enables mainly faults in channel elements and the like to be detected. Note that a configuration may also be adopted in which a channel is judged to be in error if the signal of the reflected wave is greater than a second predetermined value. Also, the member of the ultrasonic probe 10 that is used is not limited to the acoustic lens. A configuration may also be adopted in which a predetermined value is set based on the reflected intensity of an object external to the ultrasonic probe 10, and an error channel is specified based on the reflected wave from the object.

Also, for example, although, in the above method, error channels are determined relatively using data for a period corresponding to the reflected wave from the acoustic lens, a configuration may be adopted in which the reception signals (1 to N) of all the channels are used. That is, the total value $S_m$ in equation (1) can be derived for n = 1 to N, and the reference deviation $\sigma$ can be derived based thereon. With this method, if a reception wave does not reach a channel correctly due to the influence of air bubbles or the like, the channel that was not able to properly receive the ultrasonic wave can be detected as an error channel. It should be obvious that faults in channel elements can also be detected.

Next, the MVB processing unit 132 will be described in detail.

The MVB processing unit 132 performs MVB processing, which is directionally-constrained adaptive beamforming. Adaptive beamforming is processing that involves dynamically changing the sensitivity characteristics so as to not have sensitivity to unwanted waves, by changing the weight of each channel according to the incoming wave. Even if an ultrasonic beam is transmitted so as to have high sound pressure in a frontal direction, ultrasonic waves also reach reflectors that exist in directions other than directly in front, since ultrasonic waves are characterized by spreading spherically. When unwanted waves reflected by reflectors other than the target are received, azimuth resolution deteriorates due to the influence of the unwanted waves. In contrast, adaptive beamforming places a constraint on direction so as to not have sensitivity to unwanted waves, thus enabling the problem of a decrease in azimuth resolution due to unwanted waves to be remedied.

In the present embodiment, the MVB processing unit 132 performs MVB processing on normal channels other than error channels detected by the error detection unit 131. In MVB processing, the total number of normal channels excluding error channels among the M channels of the use aperture is given as M_fix channels. Also, the reception signal of the mth (1 to M_fix) channel is expressed as x_fix$_m$, and the reception signal of the nth sampling point (1 to N) in the mth channel is expressed as x_fix$_m$[n].

The reception focus processing unit 1321 provides a corresponding delay time D$_m$ determined in advance to the signal received by each normal channel so that the signals received by the respective normal channels are in phase. Since the reflected wave from a given reflector spreads spherically, a delay time is provided so that the arrival time at each vibrator is the same, and the reflected waves are added together taking into account the delay time.

An output signal X$_m$ of the mth normal channel is derived with equation (4). Also, the output signal of each normal channel is given by equation (5) when expressed in vector notation.

$$X_m = x_m[n - D_m[n]] \quad \cdots \quad (4)$$

$$\mathbf{X}[n] = \begin{bmatrix} x_1[n - D_1[n]] \\ x_2[n - D_2[n]] \\ \vdots \\ x_{M\_fix}[n - D_{M\_fix}[n]] \end{bmatrix} \quad \cdots \quad (5)$$

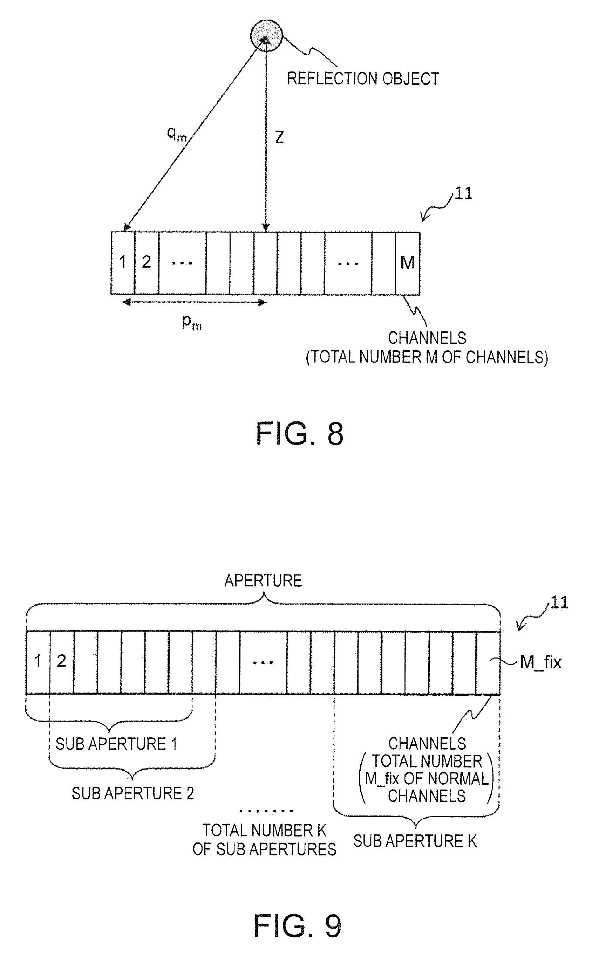

As shown in Fig. 8, the ultrasonic wave reflected from a reflection object (object) that is located in a depth direction Z from the ultrasonic transducer device 11 arrives at each channel as a spherical wave. Accordingly, the time taken for the reflection signal to arrive at the element of each channel is determined by a linear distance q$_m$ from the reflection object to the channel, with the ultrasonic wave taking longer to arrive as the distance of the element from the reflection object increases. An arrival time D'$_m$ for each element is geometrically derived as shown in equation (6), and is determined by a position p$_m$ of the ultrasonic transducer element 12 and a depth distance Z. c is the sound velocity (fixed value). Note that m is 1 to M in the description of the diagram.

$$q_m = \sqrt{p_m{}^2 + Z^2}$$
$$D'_m = q_m / c \quad \cdots \quad (6)$$

The output signal of each normal channel computed by the reception focus processing unit 1321 is output to the spatial averaging processing unit 1322.

The spatial averaging processing unit 1322 extracts a plurality of sub apertures from the aperture constituted by the M_fix normal channels, and performs processing for taking respective averages thereof. Spatial averaging is performed in order to prevent azimuth estimation accuracy from deteriorating due to the influence of correlated interference waves when the value of each channel is used directly.

For example, consider the case where K sub apertures (K = M_fix - S + 1) each consisting of S channels are extracted from the aperture of a total number M_fix of normal channels, as shown in Fig. 9. In this case, the input vector of each sth sub aperture can be represented as shown in equation (7).

$$\tilde{\mathbf{x}}\_\mathbf{fix}_s[n] = \begin{bmatrix} x\_fix_s[n - D_s[n]] \\ x\_fix_{s+1}[n - D_{s+1}[n]] \\ \vdots \\ x\_fix_{s+S-1}[n - D_s[n]] \end{bmatrix} \quad \cdots \ (7)$$

Note that, instead of spatial averaging, processing known as temporal averaging that takes the average in a time direction of each channel may be performed. Signals processed by the spatial averaging processing unit 1322 are output to the weight calculation unit 1323 or the weighted addition unit 1324.

Note that the spatial averaging processing unit 1322 is not an essential constituent element. In the case where spatial averaging is not performed, signals processed by the reception focus processing unit 1321 can be output to the weight calculation unit 1323 or the weighted addition unit 1324.

The weight calculation unit 1323 computes the weight to be applied to the output of each normal channel. Here, weight calculation will be described.

First, the case where spatial averaging is not used will be described. An output z that is output by the weighted addition unit 1324 is the result of multiplying a weight $w_m$ of each normal channel and a signal $x\_fix_m$ obtained from delay processing performed on each channel that is output from the reception focus processing unit 1321 and summing the multiplication results, and is represented by equation (8).

$$z[n] = \sum_{m=1}^{M\_fix} w_m[n] x\_fix_m[n - D_m[n]] \quad \cdots \ (8)$$

This is given by equations (9) and (10) when expressed in vector notation. H is a complex conjugate transpose and * is a complex conjugate.

$$z[n] = \mathbf{w}[n]^H \mathbf{X\_FIX}[n] \quad \cdots \ (9)$$

$$\mathbf{w}[n] = \begin{bmatrix} w_1^*[n] \\ w_2^*[n] \\ \vdots \\ w_{M\_fix}^*[n] \end{bmatrix} \quad \cdots \ (10)$$

A correlation matrix R is given by equations (11) and (12).

$$\mathbf{R}[n] = E[\mathbf{X\_FIX}[n]\mathbf{X\_FIX}[n]^T] \quad \cdots \ (11)$$

$$E\left[\|z[n]\|^2\right] = \mathbf{w}[n]^H \mathbf{R}[n]\mathbf{w}[n] \qquad \cdots \quad (12)$$

In order to compute a weight that minimizes the variance of z[n] in equations (11) and (12), conditional minimization problems such as shown in equations (13) and (14) are solved to derive the weight as shown in equation (15).

$$\min_{w[n]} \mathbf{w}[n]^H \mathbf{R}[n]\mathbf{w}[n] \qquad \cdots \quad (13)$$

$$subject\ to\ \mathbf{w}[n]^H \mathbf{a} = 1 \quad \cdots \quad (14)$$

$$w[n] = \frac{\mathbf{R}[n]^{-1}\mathbf{a}}{\mathbf{a}^H \mathbf{R}[n]^{-1}\mathbf{a}} \qquad \cdots \quad (15)$$

Here, a is a steering vector. In the present embodiment, phasing has already being performed, so the direction is 0 degrees. Accordingly, a can be set to 1.

The weighted addition unit 1324 performs weighted addition using the weight of each normal channel computed by the weight calculation unit 1323, and the reception signal of each normal channel computed by the reception focus processing unit 1321. That is, an operation using equation (8) is performed to obtain the output z. The signal computed by the weighted addition unit 1324 is output to the detection processing unit 133.

Next, the case where spatial averaging is used will be described. In this case, the correlation matrix can be expressed as shown in equation (16).

$$\tilde{\mathbf{R}}[n] = \frac{1}{M\_FIX - S + 1} \sum_{s=1}^{M\_FIX - S + 1} \tilde{\mathbf{x}}\_fix_s[n]\tilde{\mathbf{x}}\_fix_s^H[n] \qquad \cdots \quad (16)$$

At this time, the optimal weight is derived by equation (17).

$$\tilde{\mathbf{w}}[n] = \frac{\tilde{\mathbf{R}}[n]^{-1}\mathbf{a}}{\mathbf{a}^H \tilde{\mathbf{R}}[n]^{-1}\mathbf{a}} \qquad \cdots \quad (17)$$

The weighted addition unit 1324 performs weighted addition, using the weight of each normal channel computed by the weight calculation unit 1323 and the reception signal of each normal channel computed by the spatial averaging processing unit 1322. That is, an operation using equation (18) is performed to obtain the output z. The signal obtained from the adding by the weighted addition unit 1324 is output to the detection processing unit 133.

$$z[n] = \frac{1}{M\_FIX - S + 1} \sum_{s=1}^{M\_FIX-S+1} \widetilde{\mathbf{w}}[n]^{H} \widetilde{\mathbf{x}}\_\mathbf{fix}_s[n] \qquad \cdots \quad (18)$$

Next, the flow of processing by the ultrasonic imaging apparatus will be described.

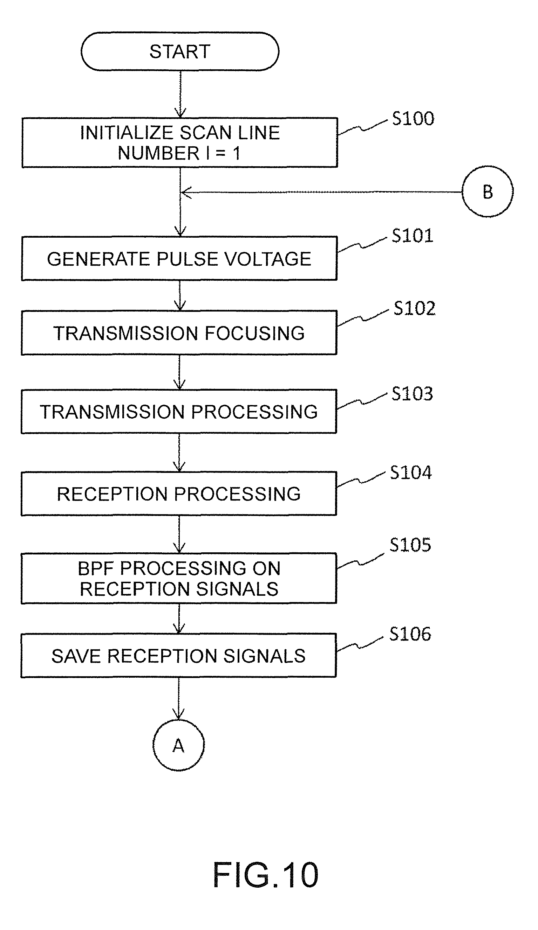

Figs. 10 to 13 are flowcharts (1 to 4) showing exemplary processing that is realized by the ultrasonic imaging apparatus. Note that the flowcharts of Figs. 10 to 13 show the flow for generating the image for one frame.

First, the control circuit 160 initializes a scan line number 1 which is a number showing the line for generating an image to 1 (1=1) (step S100). The scan line number 1 is a number showing one of the ultrasonic transducer element groups UG1 to UG64 constituting an ultrasonic transducer device such as shown in Fig. 3. For example, the scan line number 1 of an element group provided at a given edge, here, the ultrasonic transducer element group UG1, is set to "1". Also, the scan line number 1 of the element group that is adjacent to the element group having the scan line number "1", here, the ultrasonic transducer element group UG2, is set to "2". A scan line number 1 is thereby given to all the element groups. The relationship between the ultrasonic transducer element groups UG1 to UG64 and the scan line number 1 can be stored in a memory such as ROM.

Next, the control circuit 160 performs transmission and reception of an ultrasonic pulse having 0 degrees of phase at a frequency 1f, via all the channels of the use aperture corresponding to the scan line number 1 initialized at step S100 or the scan line number 1 updated at step S128 which will be discussed later (steps S101 to S106). For example, the channels of the use aperture at the time of the scan line number "1" are the ultrasonic transducer element groups UG1 to UG8, and the channels of the use aperture at the time of the scan line number "2" are the ultrasonic transducer element groups UG2 to UG9. The relationship between scan line numbers and corresponding channels of the use aperture can be stored in a memory such as ROM.

The transmission pulse generator 111 generates a pulse voltage for transmitting an ultrasonic pulse having 0 degrees of phase at a frequency 1f (step S101). The transmission delay circuit 113 performs transmission focusing control (step S102), and the ultrasonic probe 10 emits an ultrasonic beam corresponding to the pulse voltage generated at step S101 toward the object (step S103).

The control circuit 160 performs transmission/reception changeover processing via the transmission/reception changeover switch 140. The ultrasonic probe 10 receives the reception waves that come back as a result of the emitted ultrasonic beam being reflected by the acoustic lens and the object with all the channels of the use aperture, and passes the received signals to the reception processing unit 120. The reception circuit 121 converts the reception wave (analog signal) for each channel of the use aperture into a digital reception signal, and outputs the reception signals to the filter circuit 123 (step S104).

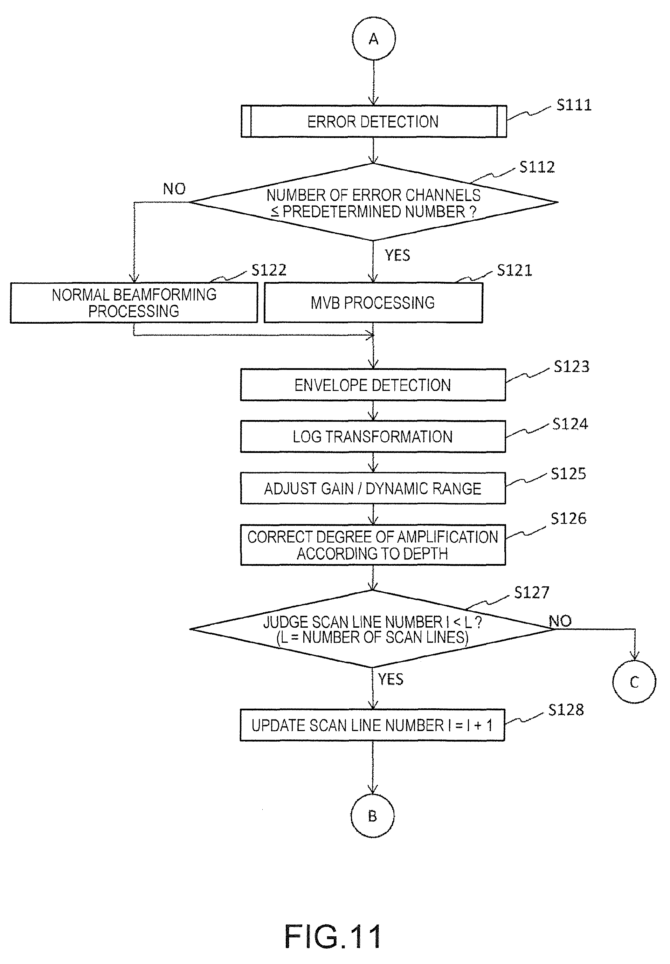

The filter circuit 123 performs bandpass filtering on the reception signal for each channel of the use aperture (step S105). The control circuit 160 saves the reception signal each channel of the use aperture output from the filter circuit 123 to the memory 125 (step S106). The processing then advances to step S111 (Fig. 11).

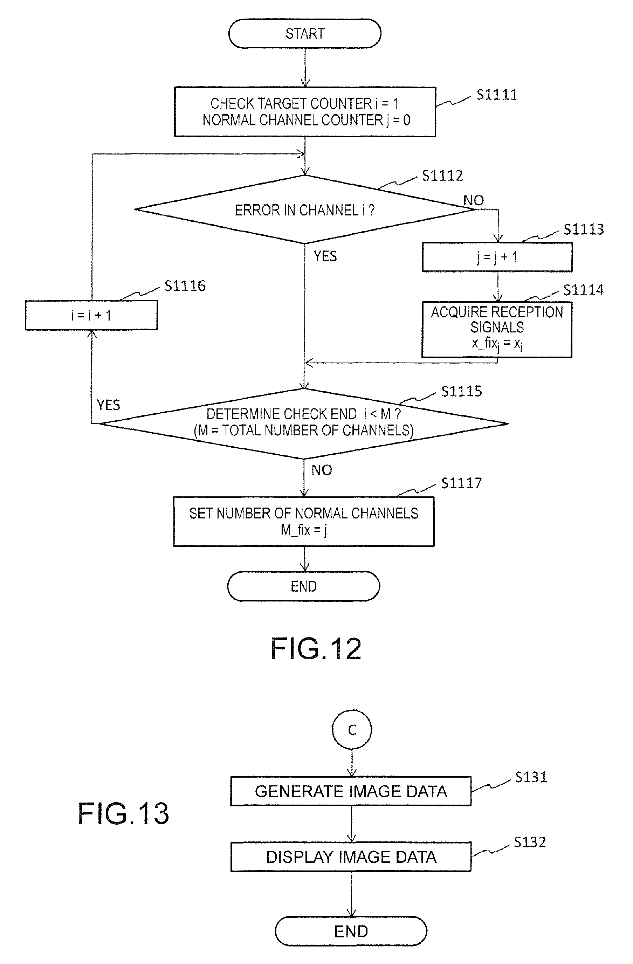

Next, the control circuit 160 issues an instruction to the error detection unit 131, and the error detection unit 131, for each channel of the use aperture, acquires the reception signal saved in the memory 125 at step S106 and performs error detection based on the acquired reception signal (step S111). The processing of step S111 will be described with reference to Fig. 12.

First, the error detection unit 131 set a counter i showing the channel number to be checked to 1, and sets a counter j showing the number of normal channels to 0 (step S1111). Note that the counter i shows each of the M channels of the use aperture corresponding to the scan line number 1.

Thereafter, the error detection unit 131 determines whether the channel corresponding to the counter i initialized at step S1111 or the counter i updated at step S1116 which will be discussed later is in error (step S1112). The details of the error detection method are as described above.

When the channel i is not an error channel (NO at step S1112), the error detection unit 131 adds 1 to the counter j to update the counter j (step S1113). Also, the error detection unit 131 acquires data $x_i$ of the reception signal of the channel i, and stores the data $x_i$ as data $x\_fix_j$ of the reception signal of a normal channel in a memory such as RAM (step S1114).

If the channel i is in error (YES at step S1112) or after step S1114, the error detection unit 131 judges whether the counter i showing the channel number to be checked is less than the total number M of channels (step S1115).

If the counter i is less than the total number M of channels (YES at step S1115), the error detection unit 131 adds 1 to the counter i to update the counter i(step S1116). The processing then returns to step S1112.

On the other hand, if the counter i is not less than the total number M of channels (NO at step S1115), that is, if the counter i coincides with the total number M of channels, the error detection unit 131 sets the number M_fix of normal channels to the value of the counter j (step S1117). The error detection unit 131 then advances the processing to step S112 (Fig. 11).

Returning to the description of Fig. 11, the MVB processing unit 132 determines whether the number of error channels is less than or equal to a predetermined number (step S112). Specifically, the MVB processing unit 132 calculates the difference (number of error channels) between the total number M of channels of the use aperture and the number M_fix of normal channels set at step S1117 (Fig. 12). It is then determined whether the number of error channels is less than or equal to a predetermined number. The predetermined number is, for example, preset to a value that allows the effects of adaptive beamforming to be sufficiently obtained, and stored in a memory such as ROM.

If the number of error channels is less than or equal to the predetermined number (YES at step S112), the MVB processing unit 132 performs MVB processing, which is directionally-constrained adaptive beamforming, based on the reception signal for each normal channel acquired at step S1114 (Fig. 12) among all the channels of the use aperture (step S121). The details of this processing are as described above. That is, the reception focus processing unit 1321 performs predetermined delay processing, for each normal channel, on the reception signal of the normal channel, and the spatial averaging processing unit 1322 performs spatial averaging on the signals that have undergone delay processing by the reception focus processing unit 1321. The weight calculation unit 1323 then computes the weight for each normal channel, and the weighted addition unit 1324 performs weighted addition of the signal of the normal channel using the computed weight.

On the other hand, if the number of error channels exceeds the predetermined number (NO at step S112), the MVB processing unit 132 performs normal beamforming processing, based on the reception signals of all the channels of the use aperture (step S122). Specifically, the reception focus processing unit 1321 performs predetermined delay processing, for each channel, on the reception signal of the channel, and outputs the processed signals to the weighted addition unit 1324. The weighted addition unit 1324 performs weighted addition of the signal of each channel using a fixed weight defined in advance for the channel.

Next, the detection processing unit 133 performs envelope detection, which involves applying a low-pass filter to the output signal for the scan line number 1 output by the MVB processing unit 132 at step S121 or step S122, after absolute value (rectification) processing has been performed thereon, and extracting an unmodulated signal (step S123). Then, the logarithmic transformation unit 135 performs logarithmic transformation (step S124).

Thereafter, the gain and dynamic range adjustment unit 137 adjusts the signal strength and the area of interest (step S125). Then, the STC 139 corrects the degree of amplification (brightness) according to depth (step S126).

Next, the control circuit 160 judges whether the scan line number 1, which indicates the line for generating an image, is less than the number L of scan lines (step S127). The number L of scan lines, in the case of an ultrasonic transducer device such as shown in Fig. 3, depends on the number of ultrasonic transducer element groups UG1 to UG64.

If the scan line number 1 is less than the number L of scan lines (YES at step S127), the control circuit 160 adds "1" to the scan line number 1 to update the scan line number 1 (step S128). The processing then returns to step S101 (Fig. 10).

On the other hand, if the scan line number 1 is not less than the number L of scan lines (NO at step S127), that is, if the scan line number 1 coincides with the number L of scan lines, the control circuit 160 issues an instruction to the image processing unit 130, and the image processing unit 130 generates a frame image from the signals of all the scan lines processed at steps S121 to S126, and advances the processing to step S131 (Fig. 13).

The DSC 150 performs scan conversion, based on the signals (frame images) of all the scan lines generated by the image processing unit 130 to generate B-mode image data (image data for display), and outputs the generated image data for display to the display unit 21 (step S131). The display unit 21 displays the generated image data for display (step S132). This ends processing of the flowchart shown in Figs. 10 to 13.

An embodiment of the invention has been described above. According to this embodiment, a decrease in the effects of adaptive beamforming can be prevented, even in the case where some of the elements cannot correctly receive signals.

That is, in the present embodiment, error channels are detected and adaptive beamforming is executed on the reception signals of normal channels other than the error channels. The influence of the reception signals of channels that cannot receive signals correctly is thereby avoided, and the likelihood of a decrease in the effects of adaptive beamforming (high azimuth resolution, etc.) can be reduced.

Also, in the present embodiment, error channel detection is performed when generating the image for one line. Faults or the like that occurs due to aged deterioration of the elements or the like can thereby be accurately detected. Also, since adaptive beamforming is performed on the reception signals of normal channels for each line, the likelihood of a decrease in the effects of adaptive beamforming can be reduced.

Also, in the present embodiment, error channels are detected relatively, using the values of the reception signals of a plurality of channels. Error channels can thereby be accurately detected, even when aged deterioration or the like occurs in all the channels.

Also, in the present embodiment, error channel detection is performed using the reflected wave of a specific member provided in the probe such as the acoustic lens. Error channels can thereby be accurately detected, without being affected by the external environment or the like.

Also, in the present embodiment, normal beamforming is performed instead of adaptive beamforming, in the case where there are more than a predetermined number of error channels. Resolution can thereby be improved by executing normal beamforming, in the case where resolution would conversely decrease if adaptive beamforming were executed.

Although the invention has been described above using embodiments, the technical scope of the invention is not limited to the scope given in the above embodiments. A person skilled in the art will appreciate that numerous changes and modifications can be made to the embodiments. Also, it is obvious from the claims that configurations to which changes and modifications are made are included in the technical scope of the invention. Also, the invention is not limited to an ultrasonic imaging apparatus, and can also be provided in various forms, such as an image processing method that is performed in an ultrasonic imaging apparatus, a program of an ultrasonic imaging apparatus, or a storage medium on which the program is stored. The invention can also be provided as an ultrasonic measurement apparatus in which the ultrasonic measurement apparatus main unit does not have the display unit 21, and generated image data for display is output to an external display unit. Also, the invention can be provided in various forms, such as an image processing method of an ultrasonic measurement apparatus, a program of an ultrasonic measurement apparatus, or a storage medium on which the program is stored. Note that, in the invention, an ultrasonic imaging apparatus main unit that does not include an ultrasonic probe may be referred to as an ultrasonic imaging apparatus, and an ultrasonic measurement apparatus main unit that does not include an ultrasonic probe may be referred to as an ultrasonic measurement apparatus.

Note that the invention can be applied with regard to any scanning method, such as linear scanning for dividing the aperture of the probe and generating lines using sub apertures, sector scanning for angling the ultrasonic beam by adjusting the delay time of each channel, and offset sector scanning for use in a convex probe. Also, the invention can even be applied with regard a scanning method in which transmission and reception are not performed every line, such as the synthetic aperture method.

## Claims

1. An ultrasonic measurement apparatus comprising:

   a reception processing unit that receives, via an ultrasonic element array having a plurality of channels, an ultrasonic echo relating to an ultrasonic wave transmitted toward an object, as a reception signal for each channel;
   an error detection unit that performs error detection for each channel;
   a signal processing unit that performs weighted addition of a reception signal for each normal channel other than an error channel for which an error was detected, with a weight that depends on the reception signal for the normal channel; and
   an image generation unit that generates an image based on a signal obtained from the weighted addition.

2. The ultrasonic measurement apparatus according to claim 1,
   wherein the reception processing unit receives an ultrasonic echo relating to the transmitted ultrasonic wave as the reception signal for each channel, whenever an ultrasonic wave for generating an image for one line is transmitted,
   the error detection unit performs error detection for each channel, whenever the reception signal for each channel is received,
   the signal processing unit performs weighted addition, whenever the reception signal for each channel is received, and
   the image generation unit generates an image, whenever the reception signal for each channel is received.

3. The ultrasonic measurement apparatus according to claim 1,
   wherein the error detection unit detects a channel whose reception signal has a relatively low value as an error channel, based on a value of the received reception signal for each channel.

4. The ultrasonic measurement apparatus according to claim 3,
   wherein the error detection unit detects a channel whose reception signal has a relatively low value as an error channel, based on a value of a reflection signal from a specific object in an ultrasonic probe that includes the ultrasonic element array, among the received reception signals for the respective channels.

5. The ultrasonic measurement apparatus according to claim 1,
   wherein the error detection unit detects a channel whose reception signal has a value less than a predetermined value as an error channel, based on a value of the received reception signal for each channel.

6. The ultrasonic measurement apparatus according to claim 1,
wherein the signal processing unit determines whether the number of the detected error channels is less than or equal to a predetermined number, performs weighted addition of the reception signal for each normal channel with the weight that depends on the reception signal for the normal channel, if the number of detected error channels is less than or equal to the predetermined number, and performs weighted addition of the reception signal for each error channel and each normal channel with a predetermined fixed weight, if the number of detected error channels is not less than or equal to the predetermined number.

7. The ultrasonic measurement apparatus according to claim 1,
wherein the signal processing unit derives, as the weight that depends on the reception signal, a weight of each normal channel at which a variance of a value obtained by multiplying a weight of the normal channel and the reception signal of the normal channel is minimized.

8. An ultrasonic imaging apparatus comprising:

a reception processing unit that receives, via an ultrasonic element array having a plurality of channels, an ultrasonic echo relating to an ultrasonic wave transmitted toward an object, as a reception signal for each channel;
an error detection unit that performs error detection for each channel;
a signal processing unit that performs weighted addition of a reception signal for each normal channel other than an error channel for which an error was detected, with a weight that depends on the reception signal for the normal channel;
an image generation unit that generates an image based on a signal obtained from the weighted addition; and
a display unit that displays the generated image.

9. An ultrasonic measurement method comprising:

receiving, via an ultrasonic element array having a plurality of channels, an ultrasonic echo relating to an ultrasonic wave transmitted toward an object, as a reception signal for each channel;
performing error detection for each channel;
performing weighted addition of a reception signal for each normal channel other than an error channel for which an error was detected, with a weight that depends on the reception signal for the normal channel; and
generating an image based on a signal obtained from the weighted addition.

FIG. 1

FIG. 2A

FIG. 2B

FIG. 2C

EP 2 846 170 A1

D2 (SCAN DIRECTION)

D1
(SLICE DIRECTION)

11

ULTRASONIC TRANSDUCER DEVICE

| CL1 | UG1 | UG2 | UG3 | UG4 | UG5 | UG6 | UG7 | UG8 | UG64 |

CL2

CL3

CL4

CL5

CL6

CL7

CL8

. . . . . .

VCOM

| DL1 | DL2 | DL3 | DL4 | DL5 | DL6 | DL7 | DL8 | DL64 |

| VT1 | VT2 | VT3 | VT4 | VT5 | VT6 | VT7 | VT8 | . . . . . . | VT64 |
| VR1 | VR2 | VR3 | VR4 | VR5 | VR6 | VR7 | VR8 | . . . . . . | VR64 |

FIG. 3

FIG. 4A          FIG. 4B

FIG. 5

FIG. 6

FIG. 7

FIG. 8

FIG. 9

START

INITIALIZE SCAN LINE
NUMBER I = 1 — S100

B

GENERATE PULSE VOLTAGE — S101

TRANSMISSION FOCUSING — S102

TRANSMISSION PROCESSING — S103

RECEPTION PROCESSING — S104

BPF PROCESSING ON
RECEPTION SIGNALS — S105

SAVE RECEPTION SIGNALS — S106

A

FIG.10

FIG.11

START

CHECK TARGET COUNTER i = 1
NORMAL CHANNEL COUNTER j = 0 — S1111

ERROR IN CHANNEL i ? — S1112

NO

j = j + 1 — S1113

ACQUIRE RECEPTION
SIGNALS
$x\_fix_j = x_i$ — S1114

i = i + 1 — S1116

YES

DETERMINE CHECK END i < M ?
(M = TOTAL NUMBER OF CHANNELS) — S1115

NO

SET NUMBER OF NORMAL CHANNELS
M_fix = j — S1117

END

YES

FIG.12

C

GENERATE IMAGE DATA — S131

DISPLAY IMAGE DATA — S132

END

FIG.13

**Europäisches Patentamt**
**European Patent Office**
**Office européen des brevets**

# EUROPEAN SEARCH REPORT

Application Number

EP 14 18 3898

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y<br>A | US 2013/194891 A1 (KRISTOFFERSEN KJELL [NO] ET AL) 1 August 2013 (2013-08-01)<br>* abstract; figures 1, 2, 6 *<br>* paragraph [0020] - paragraph [0030] *<br>* paragraph [0033] - paragraph [0035] *<br>* paragraph [0060] - paragraph [0062] * | 1-3,5, 7-9<br>4<br>6 | INV.<br>G01S7/52<br>G01S15/89 |
| X<br>Y<br>A | EP 0 713 102 A1 (ADVANCED TECH LAB [US]) 22 May 1996 (1996-05-22)<br>* abstract; figures 1, 5, 6, 7a, 7b, 7c *<br>* column 2, line 34 - column 4, line 21 *<br>* column 5, line 15 - column 7, line 47 * | 1,3-5, 7-9<br>4<br>6 | |
| A | JAMES S HALL ET AL: "Minimum variance ultrasonic imaging applied to an in situ sparse guided wave array", IEEE TRANSACTIONS ON ULTRASONICS, FERROELECTRICS AND FREQUENCY CONTROL, vol. 57, no. 10, 1 October 2010 (2010-10-01), pages 2311-2323, XP011319234, ISSN: 0885-3010<br>* chapter IV * | 1-9 | |
| A | J-F SYNNEVAG ET AL: "Adaptive Beamforming Applied to Medical Ultrasound Imaging", IEEE TRANSACTIONS ON ULTRASONICS, FERROELECTRICS AND FREQUENCY CONTROL, IEEE, US, vol. 54, no. 8, 1 August 2007 (2007-08-01), pages 1606-1613, XP011190325, ISSN: 0885-3010, DOI: 10.1109/TUFFC.2007.431<br>* chapter II * | 1-9 | |

**TECHNICAL FIELDS SEARCHED (IPC)**

G01S
A61B

The present search report has been drawn up for all claims

| Place of search | Date of completion of the search | Examiner |
|---|---|---|
| Munich | 23 January 2015 | Knoll, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 14 18 3898

This annex lists the patent family members relating to the patent documents cited in the above-mentioned European search report.
The members are as contained in the European Patent Office EDP file on
The European Patent Office is in no way liable for these particulars which are merely given for the purpose of information.

23-01-2015

| Patent document cited in search report | | Publication date | Patent family member(s) | | Publication date |
|---|---|---|---|---|---|
| US 2013194891 | A1 | 01-08-2013 | CN | 103284754 A | 11-09-2013 |
| | | | FR | 2986145 A1 | 02-08-2013 |
| | | | JP | 2013154169 A | 15-08-2013 |
| | | | US | 2013194891 A1 | 01-08-2013 |
| EP 0713102 | A1 | 22-05-1996 | EP | 0713102 A1 | 22-05-1996 |
| | | | JP | H08238243 A | 17-09-1996 |
| | | | US | 5517994 A | 21-05-1996 |

EPO FORM P0459

For more details about this annex : see Official Journal of the European Patent Office, No. 12/82

**EP 2 846 170 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2012170826 A